# EUROPEAN PATENT SPECIFICATION

(11) **EP 0 487 990 B1**
(45) Date of publication and mention of the grant of the patent: **11.10.1995**
(21) Application number: 91119492.6
(22) Date of filing: 15.11.1991
(51) Int. Cl.: C07C 49/513, C07C 45/30, C07C 45/64

(54) **Process for the preparation of a substituted indene**
Verfahren zur Herstellung von einem substituierten Inden
Procédé pour la préparation d'un indène substitué

(30) Priority: 27.11.1990 US 619167
(43) Date of publication of application: 03.06.1992
(73) Proprietor: F. HOFFMANN-LA ROCHE AG, 4002 Basel (CH)
(72) Inventor: Uskokovic, Milan Radoje, Upper Montclair, NJ 07043 (US)
(74) Representative: Grossner, Lutz, Dr.

(56) References cited:
- FR-A- 2 633 613
- TETRAHEDRON LETTERS. vol. 32, no. 43, 1991, OXFORD GB pages 6057 - 6060; J.KIEGIEL ET AL.: 'Chemical Conversion of Vitamin D3 to its 1,25-Dihydroxy Metabolite.'
- TETRAHEDRON LETTERS. vol. 30, no. 39, 1989, OXFORD GB pages 5271 - 5274; A.TENAGLIA ET AL.: 'Ruthenium-Catalyzed C-H Bond Activation Oxidation of Bridged Bicyclic and Tricyclic Alkanes.'

## Description

The invention relates to a process for the preparation of [1R-(1β(R*),3aα, 4β,7aβ)]octahydro-1-(5-hydroxy-1,5-dimethylhexyl)-7a-methyl-4H-inden-4-one, characterized by the formula 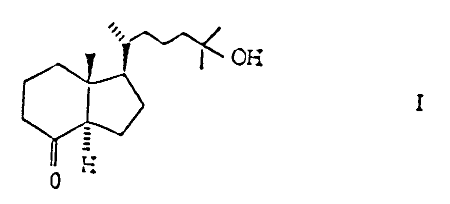
The compound of formula I is known precursor for the preparation of 1α,25-dihydroxycholecalciferol and thus is useful in the synthesis of 1α,25-dihydroxycholecalciferol, see for example, J. Org. Chem., Vol. 51, No. 16, 3100 - 3101, 1986, wherein a multistep process for the preparation of that compound is disclosed.

More particulary, the invention relates to a process for the preparation of the compound of formula I which comprises reacting [1R-(1β(R*),3aα, 7aβ]octahydro-1-(1,5-dimethylhexyl)-7a-methyl-4H-inden-4-ol, characterized by the formula 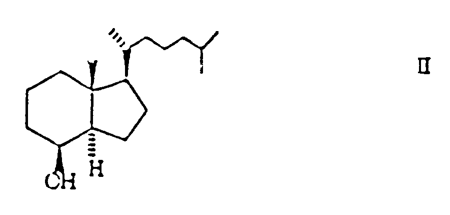
or [1R-(1β(R*),3aα,7aβ)]octahydro-1-(1,5-dimethylhexyl)-7a-methyl-4H-inden-4-one, characterized by the formula 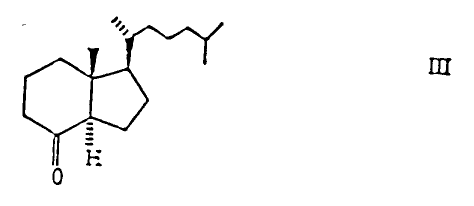
with sodium metaperiodate and ruthenium chloride hydrate and isolating the compound of formula I from the reaction mixture.

In the formulas represented herein, when substituents are illustrated as joined to the nucleus a solid line ( 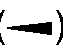 ), indicates that the substituent is in the β-orientation, that is, above the plane of the molecule, a broken line ( 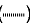 ), indicates that the substituent is in the α-orientation, that is, below the plane of the molecule.

In accordance with one aspect of the invention, the alcohol of formula II is reacted with ruthenium(III)chloride hydrate and sodium metaperiodate to yield compounds of formulas I and III. The reaction is carried out in a mixture of a chlorinated organic solvent, for example, methylene chloride, carbon tetrachloride, or the like, and a nitrile, for example, acetonitrile, or the like, preferably, a mixture of carbon tetrachloride and acetonitrile, in the presence of a buffering agent, for example, monobasic potassium phosphate-sodium hydroxide in water, which adjusts the pH of the solution in the range of from 6 to 8, preferably to a pH of 7. The reaction is carried out at a temperature in the range of from 0° to 60°C, preferably in the range of from 40°C to 50°C, more preferably at 45°C. After stirring, water is added and the solution is extracted by conventional means, such as, for example, with methylene chloride. The extracts are dried by conventional means, for example, over magnesium sulfate and evaporated. The so-obtained mixture of compounds of formulas I and III can be separated by conventional methods, for example, by flash chromatography or the like.

The compound of formula I can also be prepared by converting the compound of formula III to the compound of formula I. According to this aspect of the invention, the compound of formula III is reacted with ruthenium(III)chloride hydrate and sodium metaperiodate.

The reaction can be carried out under conditions described above for the reaction employing the compound of formula II as the starting material.

The Examples which follow further illustrate the invention. All temperatures are given in degrees Celsius.

### Example 1

2.66 g of [1R-(1β(R*),3aα,7aβ)]octahydro-1-(1,5-dimethylhexyl)-7a-methyl-4H-inden-4-ol (prepared as described in Inhoffen et al., F. Chem. Ber. 1957, 90.664), 7.48 g of sodium metaperiodate, 0.207 g of ruthenium(III)chloride hydrate, 40 ml of carbon tetrac hloride, 40 ml acetonitrile and 52 ml pH 7.0 buffer (monobasic potassium phosphate-sodium hydroxide, 0.05M in water) were stirred vigorously for 68 hours at 45°C. After this period, 30 ml water was added and the mixture extracted with 2 x 40 ml of methylene chloride. The extracts were combined, dried over magnesium sulfate and evaporated. Flash chromatography (hexane - EtOAc = 20:1, later 4:1) afforded 0.73 g (26%) of [1R-(1β(R*),3aα,7aβ)]octahydro-1-(1,5-dimethylhexyl)-7a-methyl-4H-inden-4-one (III), and 1.15 g (41%) of [1R-(1β(R*),3aα, 4β,7aβ)]octahydro-1-(5-hydroxy-1,5-dimethylhexyl)-7a-methyl-4H-inden-4-one (I) as a pale yellow oil which solidified upon standing in the freezer.

### Example 2

23.3 g of [1R-(1β(R*),3aα,7aβ)]octahydro-1-(1,5-dimethylhexyl)-7a-methyl-4H-inden-4-one, 47.11 g of sodium metaperiodate, 1.83 g of ruthenium(III)chloride hydrate, 350 ml of carbon tetrachloride, 350 ml of acetonitrile and 530 ml of pH 7.0 buffer (monobasic potassium phosphate-sodium hydroxide, 0.05M in water) were stirred vigorously for 60 hours at 45°C. After this period, 300 ml of water was added and this was extracted with 2 x 400 ml of methylene chloride. The extracts were combined, dried over magnesium sulfate and evaporated. Flash chromatography (hexaneethylacetate = 20:1, later 4:1) afforded 6.89 g (29.4%) of starting material (III) and 9.54 g (38.6%) of [1R-(1β(R*),3aα, 4β,7aβ)]octahydro-1-(5-hydroxy-1,5-dimethylhexyl)-7a-methyl-4H-inden-4-one (I) as a pale yellow oil which solidifies upon standing in the freezer.

## Claims

1. A process for the preparation of [1R-(1β(R*),3aα,4β,7aβ)]octahydro-1-(5-hydroxy-1,5-dimethylhexyl)-7a-methyl-4H-inden-4-one (I) which comprises reacting [1R-(1β(R*),3aα,7aβ)]octahydro-1-(1,5-dimethylhexyl)-7a-methyl-4H-inden-4-ol or [1R-(1β(R*), 3aα,7aβ)]octahydro-1-(1,5-dimethylhexyl)-7a-methyl-4H-inden-4-one with sodium metaperiodate and ruthenium chloride hydrate in a mixture of a chlorinated solvent and a nitrile in the presence of a buffering agent at a pH in the range of from 6 to 8 and at a temperature in the range of from 0°C to 60°C and isolating the compound I from the reaction mixture.

2. A process in accordance with claim 1, wherein the buffering agent is monobasic potassium phosphate-sodium hydroxide.

## Patentansprüche

1. Verfahren zur Herstellung des [1R-(1β(R*),3aα,4β,7aβ)]octahydro-1-(5-hydroxy-1,5-dimethylhexyl)-7a-methyl-4H-inden-4-ons (I), dadurch gekennzeichnet, dass das [1R-(1β(R*),3aα,7aβ)]octahydro-1-(1,5-dimethylhexyl)-7a-methyl-4H-inden-4-ol oder das [1R-(1β(R*), 3aα,7aβ]octahydro-1-(1,5-dimethylhexyl)-7a-methyl-4H-inden-4-on mit Natriummetaperiodat und Rutheniumchlorid-hydrat in einem Gemisch eines chlorierten Lösungsmittels und eines Nitrils in Gegenwart eines Puffers bei einem pH im Bereich von 6 zu 8 bei einer Temperatur im Bereich von 0°C bis 60°C umgesetzt und die Verbindung I aus dem Reaktionsgemisch abgetrennt wird.

2. Verfahren nach Anspruch 1, worin der Puffer das monobasische Kaliumphosphat-Natriumhydroxyd ist.

## Revendications

1. Procédé pour la préparation de la [1R-(1β(R*),3aα,4β,7aβ)] octahydro-1-(5-hydroxy-1,5-diméthylhexyl)-7a-méthyl-4H-indèn-4-one(I), qui comprend la réaction du [1R-(1β(R*),3aα,7aβ)]octahydro-1-(1,5-diméthylhexyl)-7a-méthyl-4H-indèn-4-ol oder das [1R-(1β(R*),3aα,7aβ)] octahydro-1-(1,5-diméthylhexyl)-7a-méthyl-4H-indèn-4-one, avec le métapériodate de sodium et le chlorure de ruthénium hydraté, dans un mélange d'un solvant chloré et d'un nitrile en présence d'un agent tampon à un pH compris dans un intervalle allant de 6 à 8 et à une température comprise dans l'intervalle allant de 0° C à 60° C et l'isolement du composé I à partir du mélange réactionnel.

2. Procédé selon la revendication 1, dans lequel l'agent tampon est le phosphate de potassium monobasique - hydroxyde de sodium.
